# EUROPEAN PATENT APPLICATION

(11) **EP 2 260 896 A1**
(43) Date of publication of application: **15.12.2010**
(21) Application number: 09727871.7
(22) Date of filing: 26.03.2009
(51) Int. Cl.: A61M 25/00, A61M 5/158

(54) **INDWELLING NEEDLE ASSEMBLY**

(30) Priority: 31.03.2008 JP 2008093546
(71) Applicant: Terumo Kabushiki Kaisha, Shibuya-ku Tokyo 151-0072 (JP)
(72) Inventor: TANABE, Hidenori, Nakakoma-gun Yamanashi 409-3853 (JP); KOBAYASHI, Ryoji, Nakakoma-gun Yamanashi 409-3853 (JP); MURASHITA, Takato, Nakakoma-gun Yamanashi 409-3853 (JP)
(74) Representative: TBK-Patent
(86) International application number: PCT/JP2009/056190
(87) International publication number: WO 2009/123025

(57) **Abstract**

An indwelling needle assembly comprises an inner needle having a sharp needle tip at the end, an inner needle hub secured to the proximal end of the inner needle, a hollow outer needle into which the inner needle is inserted, an outer needle hub which is secured to the proximal end of the outer needle and which has a resin projection at the proximal end, and a protector which is elastic, which has a connection member with a projecting part engageable with the projection and which is detachably connected to the outer needle hub by the engagement of the projecting part with the projection. The projection of the outer needle hub comprises a sliding surface on which the projecting part is slidingly moved. The connection member can take a first state in which the projecting part is engaged with the projection and a second state in which the projecting part is disengaged from the projection. When the connection member is changed from the first state to the second state, the projecting part is slidingly moved along the sliding surface of the projection. The first contact part of the projecting part which is brought into contact with the sliding surface is formed of a resin.

## Description

### TECHNICAL FIELD

The present invention relates to an indwelling needle assembly to puncture and indwell in a blood vessel at the time of infusion, for example.

### BACKGROUND ART

At the time of carrying out an infusion on a patient or in other similar situations, an indwelling needle connected with an infusion line is made to puncture a blood vessel of the patient and is kept indwelling there during infusion.

Such an indwelling needle (indwelling needle assembly) is composed of a hollow outer needle, an outer needle hub secured to the proximal end (base end) of the outer needle, an inner needle which is inserted into the outer needle and which has a sharp needle tip at its distal end, an inner needle hub secured to the proximal end (base end) of the inner needle, and a protector detachably connected to the outer needle hub. The outer needle hub is provided with a main pipe having a flow path communicating with a lumen of the outer needle and a side pipe having a branch flow path branched from the flow path in the main pipe, and the infusion line is connected to the side pipe. (See, for example, Patent Document 1.)

When the indwelling needle punctures the patient's blood vessel, the puncturing operation is performed in the condition where the inner needle is inserted into the outer needle and the needle tip of the inner needle protrudes from the distal end of the outer needle in an assembled state.

When the needle tip of the inner needle has reached the inside of the blood vessel, blood flows into the inner needle through a distal portion thereof. The blood, in its course, flows through a hole formed in a side portion of the inner needle into a flow path between the outer needle and the inner needle, flows through the flow path, then flows into the inside of the transparent outer needle hub, specifically, into the flow path in the main pipe, and further flows through the main pipe into the branch flow path in the side pipe (flashback). Consequently, it can be confirmed (visually confirmed) that the inner needle has captured (reached securely the inside of) the blood vessel.

After this flashback is confirmed, the outer needle is advanced using the inner needle as a guide, and the outer needle is inserted into (let puncture) the blood vessel.

Next, while fixing the outer needle and the outer needle hub by grasping them with one hand, the inner needle hub is grasped with the other hand and moved in the proximal direction, whereby the inner needle is pulled out of the outer needle. Then, an infusion agent is administered through the infusion line, the side pipe and the main pipe of the outer needle hub, and the outer needle which are connected to one another.

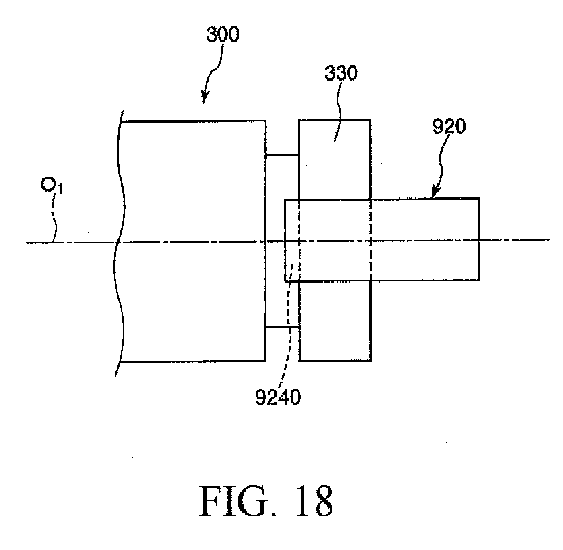

Meanwhile, as shown in FIG. 18, an outer needle hub 300 is provided with a rib 330 at its proximal portion (an end portion on the right side in FIG. 18).

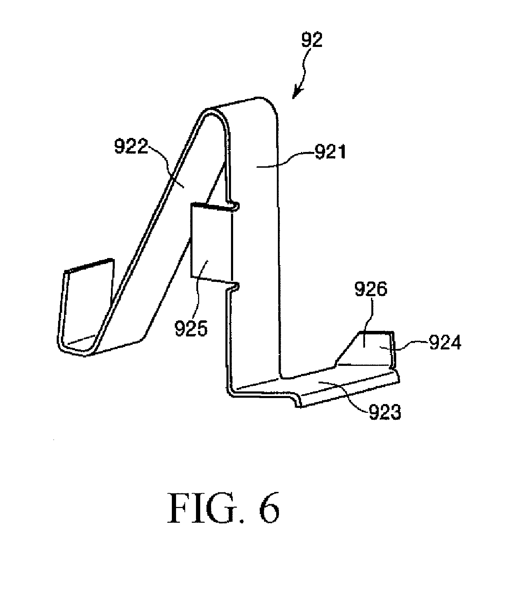

On the other hand, the protector is equipped with a V-shaped spring 920 which has a projecting piece 9240 engageable with the rib 330 of the outer needle hub 300 (see FIG. 6). Incidentally, in FIG. 18, of the spring 920, only a part inclusive of the projecting piece 9240 is shown. By the engagement of the projecting piece 9240 of the spring 920 with the rib 330 of the outer needle hub 300, the protector is detachably connected to the outer needle hub 300.

In addition, the spring 920 makes contact with an outer peripheral surface of the inner needle in the condition where the projecting piece 9240 thereof is engaged with the rib 330 of the outer needle hub 300 through elastic deformation thereof, whereby the engaged state is maintained. When the inner needle is pulled out of the outer needle, the spring 920 is restored by parting from the outer peripheral surface of the inner needle, and, at the time of the restoration of the spring 920, the projecting piece 9240 is moved toward the lower side in FIG. 18 relative to the rib 330, whereby the projecting piece 9240 and the rib 330 are disengaged from each other.

However, since the outer needle hub 300 is formed of a resin whereas the spring 920 is formed of a metal, an edge part (or a burr or the like) of the projecting piece 9240 would bite into the rib 330 when the projecting piece 9240 is moved toward the lower side in FIG. 18 relative to the rib 330. This would result in that it is difficult for the projecting piece 9240 to move, and it is difficult for the projecting piece 9240 and the rib 330 to be disengaged from each other. This constitutes a resistance in releasing the protector from the outer needle hub 300, so that the indwelling outer needle may slip off the blood vessel.

### Patent Document 1: U.S. Patent No. 6,749,588

### DISCLOSURE OF INVENTION

It is an object of the present invention to provide an indwelling needle assembly such that a protector can be smoothly released from an outer needle hub.

In order to attain the above object, the present invention provides an indwelling needle assembly including:
an inner needle having a sharp needle tip at a distal end;
an inner needle hub secured to a proximal portion of the inner needle;
a hollow outer needle into which the inner needle is inserted;
an outer needle hub which is secured to a proximal portion of the outer needle and which has a resin projection at a proximal portion thereof; and
a protector which has an elastic connection member with a projecting part engageable with the projection, and which is detachably connected to the outer needle hub by engagement of the projecting part with the projection, wherein
the projection of the outer needle hub includes a sliding surface on which the projecting part is slidingly moved,
the connection member takes a first state in which the projecting part is engaged with the projection and a second state in which the projecting part is disengaged from the projection, and, when the connection member is changed from the first state to the second state, the projecting part is slidingly moved along the sliding surface of the projection, and
a first contact part of the projecting part which is brought into contact with the sliding surface is formed of a resin.

According to the present invention as mentioned above, both the projection of the outer needle hub and the first contact part of the projecting part are each formed of a resin. Therefore, at the time of pulling the inner needle out of the outer needle and releasing the protector from the outer needle hub, the projecting part of the connection member of the protector can be slidingly moved along the sliding surface of the projection of the outer needle hub smoothly. Consequently, the operation of pulling the inner needle out of the outer needle and releasing the protector from the outer needle hub can be performed smoothly and assuredly.

In addition, in the indwelling needle assembly of the present invention, preferably, the first contact part is brought into point contact or line contact with the sliding surface.

This ensures that the area (area of contact) of the region of contact between the projecting part and the sliding surface of the projection becomes small, and the frictional resistance between the projecting part and the sliding surface of the projection is reduced. As a result, the projecting part can be slidingly moved along the sliding surface of the projection more smoothly.

Further, according to the indwelling needle assembly of the present invention, preferably, b/a is in a range from 0.3 to 3, where "a" is the Rockwell hardness of the projection and "b" is the Rockwell hardness of the first contact part.

This ensures that the projecting part of the connection member can be slidingly moved along the sliding surface of the projection more smoothly.

In addition, in the indwelling needle assembly of the present invention, preferably, the connection member is held in the first state in a state of being elastically deformed by making contact with an outer peripheral surface of the inner needle, the connection member is restored by parting from the outer peripheral surface of the inner needle when the inner needle is pulled out of the outer needle, and, when the connection member is restored, the projecting part is slidingly moved along the sliding surface of the projection, resulting in the second state.

This ensures that at the time of pulling the inner needle out of the outer needle and releasing the protector from the outer needle hub, the resistance (resistance to release) can be reduced. Consequently, the operation of pulling the inner needle out of the outer needle and releasing the protector from the outer needle hub 3 can be performed smoothly and assuredly.

Further, in the indwelling needle assembly of the present invention, preferably,
the connection member has a second contact part which makes contact with the inner needle and on which the inner needle is slidingly moved when the inner needle is pulled out of the outer needle; and
the second contact part is formed of a resin.

This permits the inner needle to be slidingly moved along the outer surface of the connection member smoothly. Accordingly, the operation of pulling the inner needle out of the outer needle and releasing the protector from the outer needle hub can be carried out smoothly and assuredly.

In addition, in the indwelling needle assembly of the present invention, preferably, the connection member has a shape obtained by bending a belt-like member, and the second contact part is secured to one outer surface of the connection member.

This ensures that, for example during an operation of releasing the protector from the outer needle hub, the second contact part is securely prevented from being released unwillingly.

Further, in the indwelling needle assembly of the present invention, preferably, the whole connection member is formed of a resin.

This ensures that at the time of pulling the inner needle out of the outer needle and releasing the protector from the outer needle hub, the projecting part of the connection member can be prevented from biting into the projection, and the projecting part can be slid (moved) along the sliding surface projection of the projection smoothly. Consequently, the operation of pulling the inner needle out of the outer needle and releasing the protector from the outer needle hub can be carried out smoothly and assuredly.

In addition, in the indwelling needle assembly of the present invention, preferably,
a direction in which the projecting part is moved by restoration of the connection member is a direction substantially perpendicular to an axis of the inner needle; and
the projection is formed at an outer peripheral surface of the outer needle hub along a circumferential direction of the outer needle hub.

This ensures that at the time of pulling the inner needle out of the outer needle and releasing the protector from the outer needle hub, the resistance (resistance to release) can be reduced. Consequently, the operation of pulling the inner needle out of the outer needle and releasing the protector from the outer needle hub 3 can be performed smoothly and assuredly.

Further, the indwelling needle assembly of the present invention, preferably, further includes connection means for connecting the inner needle or the inner needle hub with the protector, wherein
when the inner needle is pulled out of the outer needle, the inner needle hub is moved in a proximal direction, whereby the protector is moved in the proximal direction so as to be released from the outer needle hub.

This ensures that the protector is securely prevented from slipping off the needle tip of the inner needle, and, therefore, it is possible to securely maintain the condition where the needle tip is covered by the protector. Consequently, at the time of discarding the inner needle or the like or in other similar situations, the accident that the worker or the like might stick his or her finger or the like with the needle tip by mistake can be securely prevented, and high safety is ensured.

In addition, in the indwelling needle assembly of the present invention, preferably, the protector covers at least the needle tip of the inner needle when the inner needle has been pulled out of the outer needle.

This ensures that at the time of discarding the inner needle or the like or in other similar situations, the accident that the worker or the like might stick his or her finger or the like with the needle tip can be securely prevented, and high safety is promised.

### BRIEF DESCRIPTION OF DRAWINGS

FIG. 1 is a perspective view showing a first embodiment of the indwelling needle assembly according to the present invention;
FIG. 2 is an exploded perspective view of the indwelling needle assembly shown in FIG. 1;
FIG. 3 is a sectional view showing an outer needle hub, an inner needle, an inner needle hub, a protector, etc. of the indwelling needle assembly shown in FIG. 1;
FIG. 4 is a sectional view taken along line A-A of FIG. 3;
FIG. 5 is a sectional view showing the outer needle hub, the inner needle, the inner needle hub, the protector, etc. of the indwelling needle assembly shown in FIG. 1;
FIG. 6 is a perspective view showing a connection member of the protector of the indwelling needle assembly shown in FIG. 1;
FIG. 7 is a bottom view showing schematically the outer needle hub and the connection member of the protector in the indwelling needle assembly shown in FIG. 1;
FIG. 8 is a bottom view showing schematically the outer needle hub and the connection member of the protector in the indwelling needle assembly shown in FIG. 1;
FIG. 9 is a perspective view showing schematically another configuration example of the projecting piece of the connection member of the protector in the indwelling needle assembly shown in FIG. 1;
FIG. 10 is a perspective view showing schematically another configuration example of the projecting piece of the connection member of the protector in the indwelling needle assembly shown in FIG. 1;
FIG. 11 is a perspective view showing a connection member of a protector in a second embodiment of the indwelling needle assembly according to the present invention;
FIG. 12 is a perspective view showing a connection member of a protector in a third embodiment of the indwelling needle assembly according to the present invention;
FIG. 13 is an exploded perspective view showing a fourth embodiment of the indwelling needle assembly according to the present invention;
FIG. 14 is a sectional view showing an outer needle hub, an inner needle, a protector, etc. of the indwelling needle assembly shown in FIG. 13;
FIG. 15 is a sectional view showing the outer needle hub, the inner needle, the protector, etc. of the indwelling needle assembly shown in FIG. 13;
FIG. 16 is a perspective view showing a connection member of the protector in the indwelling needle assembly shown in FIG. 13;
FIG. 17 is a perspective view showing a shutter member of the protector in the indwelling needle assembly shown in FIG. 13; and
FIG. 18 is a bottom view showing schematically an outer needle hub and a connection member of a protector in a conventional indwelling needle assembly.

### BEST MODE FOR CARRYING OUT THE INVENTION

The indwelling needle assembly according to the present invention will be described in detail below, based on preferred embodiments shown in the accompanying drawings.

### <First Embodiment>

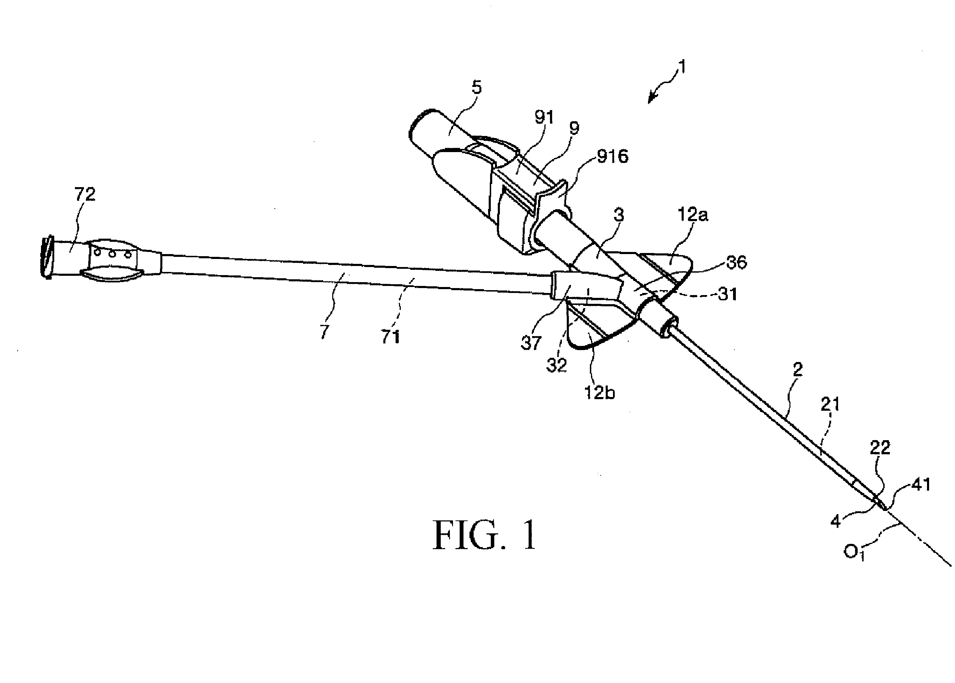

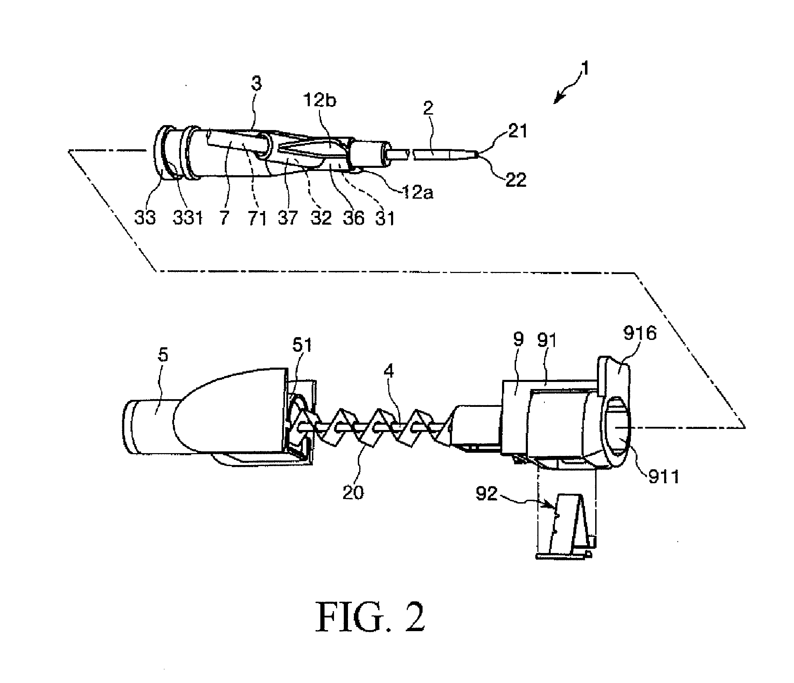

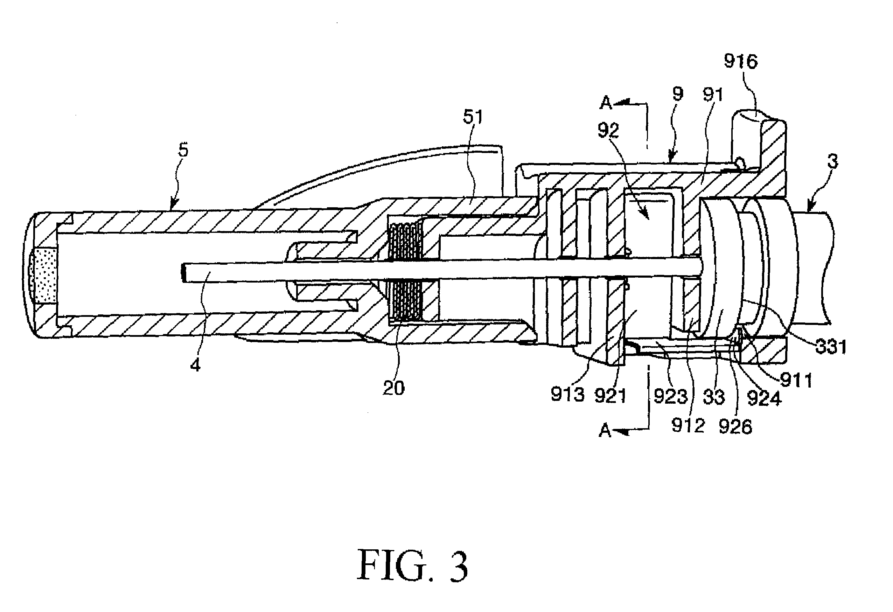

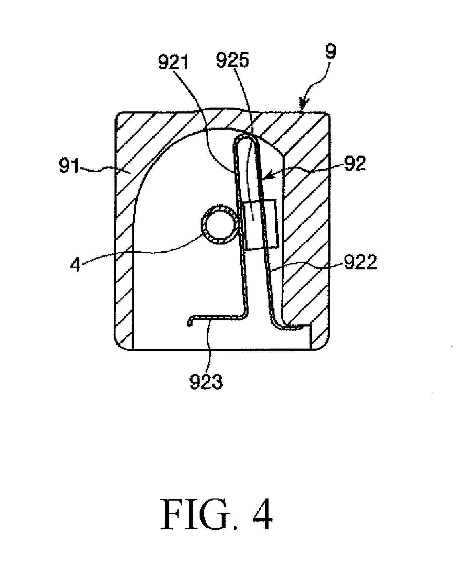

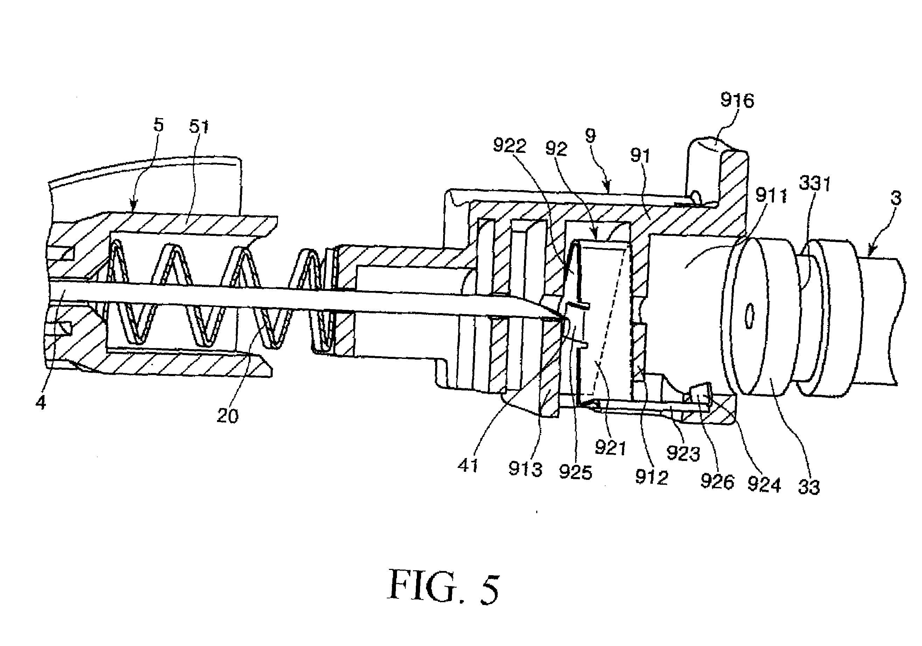

FIG. 1 is a perspective view showing a first embodiment of the indwelling needle assembly according to the present invention; FIG. 2 is an exploded perspective view of the indwelling needle assembly shown in FIG. 1; FIG. 3 is a sectional view showing an outer needle hub, an inner needle, an inner needle hub, a protector, etc. of the indwelling needle assembly shown in FIG. 1; FIG. 4 is a sectional view taken along line A-A of FIG. 3; FIG. 5 is a sectional view showing the outer needle hub, the inner needle, the inner needle hub, the protector, etc. of the indwelling needle assembly shown in FIG. 1; FIG. 6 is a perspective view showing a connection member of the protector of the indwelling needle assembly shown in FIG. 1; FIGS. 7 and 8 are each bottom views showing schematically the outer needle hub and the connection member of the protector in the indwelling needle assembly shown in FIG. 1; and FIGS. 9 and 10 are each perspective views showing schematically other configuration examples of the projecting piece of the connection member of the protector in the indwelling needle assembly shown in FIG. 1.

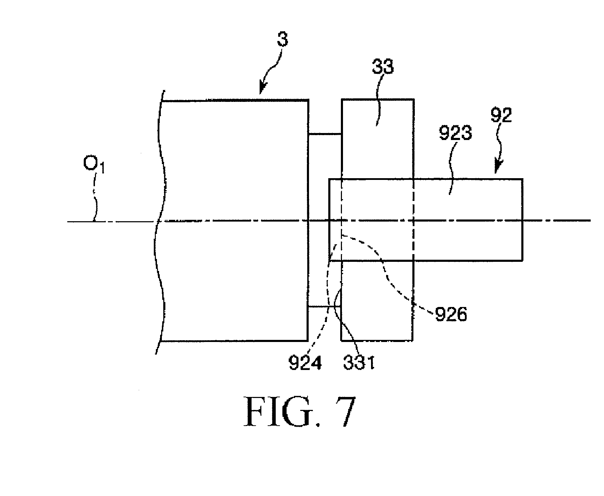

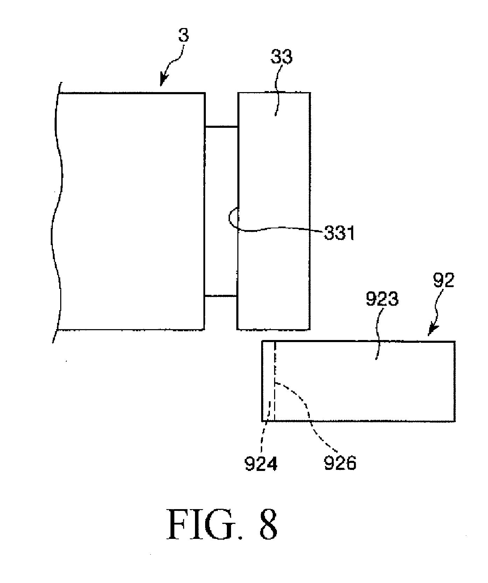

Incidentally, in the following, the left side in FIGS. 2, 3 and 5 will be referred to as a "proximal" side and the right side as a "distal" side. In addition, the right side in FIGS. 7 and 8 will be referred to as a "proximal" side and the left side as a "distal" side. Further, in FIGS. 7 and 8, of the connection member of the protector, only a first part and a projecting piece are drawn.

An indwelling needle assembly 1 shown in the drawings includes a hollow outer needle 2, an outer needle hub 3 secured to a proximal portion of the outer needle 2, an inner needle 4 inserted into the outer needle 2, an inner needle hub 5 secured to a proximal portion of the inner needle 4, and a tube 7 connected to a side portion (or a proximal portion) of the outer needle hub 3 so that its lumen 71 communicates with a lumen 21 of the outer needle 2. Configurations of the parts will be described below.

As the outer needle 2, one having a certain degree of flexibility is preferably used. The material constituting the outer needle 2 is preferably a resin material, particularly a flexible resin material. Examples of the material include fluoro-resins such as PTFE, ETFE, PFA, etc., olefin resins such as polyethylene, polypropylene, etc. and mixtures thereof, polyurethane, polyesters, polyamides, polyether nylon resins, and mixtures of the olefin resins with ethylene-vinyl acetate copolymer.

The outer needle 2 as above-mentioned may, wholly or partly, have inside visibility. Further, the material constituting the outer needle 2 may be admixed with a radiopaque material such as barium sulfate, barium carbonate, bismuth carbonate, tungstic acid, etc. so as to be radiopaque.

The outer needle hub 3 is secured (fixed) to the proximal portion of the outer needle 2 in a liquid-tight fashion by such a method as caulking, fusing (heat fusing, microwave fusing, etc.), adhesion with an adhesive, etc.

The outer needle hub 3 has a substantially tubular main pipe 36, and a side pipe (branch pipe) 37 which has a branch flow path 32 branched from a flow path 31 in the main pipe 36 and which is substantially tubular.

As has been described above, a proximal portion of the outer needle 2 is secured to the distal side of the main pipe 36, and the flow path 31 communicates with the lumen 21 of the outer needle 2 on its distal side. The flow path 31 (main pipe 36) is disposed such that its axis (center axis) substantially coincides with the center axis of the outer needle 2.

On the other hand, the branch flow path 32 (side pipe 37) is disposed such that its axis (center axis) is inclined at a predetermined angle relative to the center axis of the outer needle 2, that is, the axis of the flow path 31 (main pipe 36). In this case, the branch flow path 32 is so inclined that the proximal side of the branch flow path 32 is located on the left side in FIG. 2. Incidentally, the axis of the branch flow path 32 (side pipe 37) may be perpendicular to the axis of the flow path 31 (main pipe 36) (may be non-inclined).

In addition, a distal portion (one end portion) of the tube 7 is connected to the side pipe 37 of the outer needle hub 3. This ensures that a liquid such as a medicinal liquid (infusion liquid) can be supplied into the outer needle 2 (outer needle hub 3) by way of the tube 7.

Further, the outer needle hub 3 is formed with a rib 33 at a proximal portion thereof. The rib 33 will be detailed later.

In addition, on lateral sides of the outer needle hub 3, a pair of wings 12a and 12b are formed integrally with the outer needle hub 3. At the time of causing the outer needle 2 and the inner needle 4 to puncture a blood vessel or the like, puncturing is conducted by pinching the inner needle hub 5 by a thumb and a middle finger, and, when the distal end of the outer needle 2 has reached the inside of the blood vessel, a finger holder part 916 which will be described later is pushed by an index finger to advance the outer needle hub 3, whereby only the outer needle 2 can be advanced into the blood vessel. At the time of leaving the outer needle 2 indwelling, the wings 12a and 12b are fixed to a skin by a pressure sensitive adhesive tape or the like.

The inner needle 4 having a sharp needle tip 41 at the distal end thereof is inserted into the outer needle 2. The indwelling needle assembly 1 is used in the state wherein the inner needle 4 is inserted into the outer needle 2 and the needle tip 41 is protruded from a tip opening 22 of the outer needle 2, that is, in the state as shown in FIGS. 1, 3, 4 and 7. Hereinafter, this state will be referred to as the "assembled state."

The length of the inner needle 4 is set such that at least the needle tip 41 protrudes from the tip opening 22 of the outer needle 2 in the assembled state.

The inner needle 4 may be a hollow needle or may be a solid needle. Where the inner needle 4 is a solid needle, a sufficient strength can be secured while letting the outside diameter small. In addition, where the inner needle 4 is a solid needle, it is ensured that at the time of discarding the inner needle 4 after an operation is over, there is no risk that blood might remain inside the inner needle 4 or the blood might flow out, and, therefore, high safety is secured.

Further, where the inner needle 4 is a hollow needle, upon puncture of a blood vessel by the inner needle 4, blood flows into the hollow portion of the inner needle 4, permitting flashback of blood to be confirmed. Where the inner needle 4 is a solid needle, the flashback of blood can be confirmed earlier, since the blood flows into a gap between the inner needle 4 and the outer needle 2.

In the configurations shown in the drawings, the inner needle 4 is a hollow needle, and is formed with a hole (side hole) (not shown) in a side portion of an intermediate part thereof. This ensures that upon puncture of a blood vessel by the inner needle 4, blood flows into the hollow portion of the inner needle 4, and the blood, in its course, flows through the hole formed in the side portion of the inner needle 4 into the gap between the inner needle 4 and the outer needle 2, whereby the flashback of blood can be confirmed earlier.

Incidentally, while the inner needle 4 can have a configuration in which both a hollow portion and a solid portion are present (for example, a configuration in which a part of the lumen of a hollow needle is filled up so that the needle is hollow on the distal side and solid on the proximal side), composing the inner needle 4 wholly of a single member promises a reduction in the cost of the inner needle 4.

In addition, the inner needle 4 may be constant in outside diameter, or may have a plurality of parts differing in outside diameter.

The material constituting the inner needle 4 as described above may be a metallic material such as, for example, stainless steel, aluminum or aluminum alloys, titanium or titanium alloys, etc.

The inner needle hub 5 is secured (fixed) to a proximal portion of the inner needle 4. The inner needle hub 5 is provided with a protector insertion section (connection member containing section) 51 at its distal portion. In the protector insertion section 51, a proximal portion of a protector 9 to be described later is inserted (disposed) and a connection member 20 to be described later is contained (disposed), in the assembled state. The protector 9 and the connection member 20 can each be moved relative to the protector insertion section 51.

Examples of the method for fixing the inner needle 4 to the inner needle hub 5 include such methods as fitting, caulking, fusing, adhesion with an adhesive, etc. and combinations of some of these methods. Incidentally, in the configuration shown in the figures, the inner needle 4 is hollow, and, therefore, a proximal portion of the inner needle hub 5 is sealed so that the blood flowing back upon puncture of a blood vessel would not fly out from the proximal portion of the inner needle hub 5.

The inner needle hub 5 as just-mentioned and the outer needle hub 3 described above are each preferably formed from a transparent (colorless transparent), colored transparent or semi-transparent resin, whereby inside visibility is secured. This ensures that when the outer needle 2 has captured (has reached the inside of) a blood vessel, flashback of the blood flowing in through the inner needle 4 mentioned above can be visually confirmed.

Material constituting the inner needle hub 5 is not particularly limited. Examples of the material include various resin materials such as polyolefins such as polyethylene, polypropylene, ethylene-vinyl acetate copolymer, etc., polyurethane, polyamides, polyesters, polycarbonate, polybutadiene, polyvinyl chloride, polyacetal, etc.

In addition, the outer needle hub 3 is formed of a resin (resin material). Examples of the material constituting the outer needle hub 3 include various resin materials such as polyolefins such as polyethylene (PE), polypropylene (PP), etc., polycarbonate (PC), polyesters such as polyethylene terephthalate (PET), polybutylene terephthalate (PBT), etc., acrylic resin, ABS resin, AS resin, polyacetal (POM), etc., which may be used either singly or in combination of two or more thereof.

The tube 7 is flexible, and its distal (end) portion is connected to the side pipe 37 of the outer needle hub 3, as above-mentioned. A connector 72 is attached to a proximal portion (other end portion) of the tube 7. The connector 72 is connected with, for example, a connector attached to an end portion of an infusion line for supplying an infusion liquid (medicinal liquid) to be administered, a mouth portion (tip portion) of a syringe in which a medicinal liquid is contained, or the like.

Incidentally, the material constituting the tube 7 is not particularly limited; examples of the material include polyolefins such as polyethylene, polypropylene, ethylene-vinyl acetate copolymer, etc., polyvinyl chloride, polybutadiene, polyamides, polyurethane, polyesters, etc.

In addition, the indwelling needle assembly 1 is provided with a cylindrical (block-shaped) seal member (not shown) as sealing means for sealing the flow path 31, in the flow path 31 of the outer needle hub 3. The seal member is disposed (fixed) in the flow path 31 of the main pipe 36 on the proximal side relative to the branch flow path 32. In this embodiment, the seal member is disposed in the vicinity of the branch flow path 32.

The seal member is formed with a hole or slit into which the inner needle 4 can be inserted and which is closed when the inner needle 4 inserted thereinto is pulled out. In this embodiment, the seal member is provided with a slit (not shown) piercing the seal member along the longitudinal direction thereof, substantially in its center.

The slit is in the shape of a straight line segment. This permits the slit in a closed state to be easily brought into an open state. Therefore, the inner needle 4 can be smoothly inserted through the seal member (slit); specifically, at the time of advancing the outer needle 2 using the inner needle 4 as a guide as will be described later, frictional resistance between an outer surface of the inner needle 4 and an inner surface of the slit can be reduced. Accordingly, operability at the time of a puncturing operation of the indwelling needle assembly 1 is enhanced more.

The seal member has a self-closing property such that the inner needle 4 can be inserted into the slit in the assembled state and, when the inner needle 4 thus inserted is pulled out, the slit is closed by an elastic force (restoring force) of the seal member itself. This ensures that when the inner needle 4 is pulled out, leakage of liquid from the proximal end of the outer needle hub 3 can be prevented, and asepsis of the inside of the outer needle hub 3 can be maintained.

Examples of the material constituting the seal member as described above include various elastic materials such as various rubber materials (particularly, vulcanized ones) such as natural rubber, isoprene rubber, butyl rubber, butadiene rubber, styrene-butadiene rubber, urethane rubber, nitrile rubber, acrylic rubber, fluoro-rubber, silicone rubber, etc., various thermoplastic elastomers based on urethane, polyester, polyamide, olefin, styrene or the like, and mixtures thereof.

Further, the indwelling needle assembly 1, preferably, has been subjected to a friction reducing treatment for reducing the frictional resistance between the inner surface of the slit and the outer surface of the inner needle 4.

Examples of the friction reducing treatment include a treatment in which a lubricant is applied to at least one of the inner surface of the slit and the outer surface (outer peripheral surface) of the inner needle 4, and a treatment in which a layer composed of a low-friction material (low-friction layer) is formed on the inner surface of the slit.

Such a friction reducing treatment enables an assured reduction in the frictional resistance between the inner needle 4 and the seal member when the outer needle 2 is advanced using the inner needle 4 as a guide. This ensures that the outer needle 2 can be moved smoothly, and the indwelling needle assembly 1 is made to be excellent in operability at the time of a puncturing operation.

In addition, the indwelling needle assembly 1 has the protector 9 which covers at least the needle tip 41 of the inner needle 4 when the inner needle 4 has been pulled out of the outer needle 2. The protector 9 will be described below.

The protector 9 is inserted (disposed) in the protector insertion section 51 of the inner needle hub 5 in the assembled state.

The protector 9 is detachably connected to the outer needle hub 3. As shown in FIGS. 2, 3 and 5, the protector 9 has a protector body 91 and a connection member 92 provided inside the protector body 91.

The protector body 91 is provided with a bottomed hole 911 in its distal portion. Into the bottomed hole 911, a proximal portion of the outer needle hub 3 is inserted in the assembled state.

The material constituting the protector body 91 is not particularly limited; for example, materials identical or similar to those mentioned above as materials for the outer needle hub 3 and the inner needle hub 5 can be used.

Further, in the hole 911 in the protector body 91, the connection member 92 is contained on the proximal side. Specifically, the protector body 91 is formed with a wall section 912 constituting a bottom wall of the hole 911, and a wall section 913 disposed on the proximal side of the wall section 912, with the connection member 92 being disposed between the wall section 912 and the wall section 913.

As shown in FIGS. 3, 5 and 6, the connection member 92 has a plate-like projecting piece (projecting part) 924 which is elastic and which is engageable with the rib 33 of the outer needle hub 3. In the assembled state, the protector 9 is detachably connected to the outer needle hub 3 by the engagement of the projecting piece 924 of the connection member 92 with the rib 33 of the outer needle hub 3 in the condition where a proximal portion of the outer needle hub 3 is inserted into the hole 911 of the protector 9.

In the present embodiment, the connection member 92 as a whole has a shape (substantially V shape) obtained by bending a belt-like plate member (member) into a substantially V shape, and the plate member is elastic (is elastically deformable). Specifically, the connection member 92 includes a first part 921, a second part 922 which is connected to the upper side in FIG. 6 of the first part 921 and which forms the substantially V shape together with the first part 921, a third part 923 disposed on the lower side in FIG. 6 of the first part 921, the projecting piece 924 projecting toward the upper side in FIG. 6 from the proximal end of the third part 923, and a shutter part 925 disposed at an intermediate portion of the first part 921 and on the proximal side. In addition, a portion of the second part 922 which is on the lower side in FIG. 6 is curved or bent toward the upper side. Further, an opposed surface 926 of the projecting piece 924 opposed to a sliding surface 331 of the rib 33 to be described later is substantially perpendicular to the center axis O₁ of the inner needle 4.

The connection member 92 can take (can be deformed into) a first state (first shape) in which the projecting piece 924 and the rib 33 are engaged with each other and a second state (second state) in which the projecting piece 924 and the rib 33 are disengaged from each other, through a change in the opening angle between (mutual opening/closing of) the first part 921 and the second part 922.

Specifically, in the assembled state, as shown in FIGS. 3, 4 and 7, the connection member 92 is contained in the state of being folded to reduce the opening angle (in an elastically deformed state), and the first part 921 makes contact with an outer peripheral surface of the inner needle 4, whereby the connection member 92 is held in the first state. In this state, the protector 9 is connected to the outer needle hub 3. In addition, the protector 9 and the outer needle hub 3 are movable relative to the inner needle 4 and the inner needle hub 5 along the longitudinal direction of the inner needle 4.

When, starting from this state, the inner needle hub 5 is moved in the proximal direction relative to the protector 9 and the needle tip 41 of the inner needle 4 reaches the proximal side of the first part 921 of the connection member 92, the connection member 92 is opened (restored) by its own elastic force (restoring force), and is moved by being pulled in the proximal direction through the connection member 20 (described later), resulting in the second state, as shown in FIGS. 5 and 8. The direction in which the projecting piece 924 is moved by the restoring of the connection member 92 is a direction substantially perpendicular to the center axis O₁ of the inner needle 4. In this state, the protector 9 and the outer needle hub 3 are disconnected. In addition, the shutter part 925 of the connection member 92 is located on the distal side of the needle tip 41 on the center axis O₁ of the inner needle 4, whereby the needle tip 41 is inhibited from moving (passing) in the distal direction past (beyond) the connection member 92. Incidentally, when the inner needle hub 5 is moved further in the proximal direction, the protector 9 is moved by being pulled in the proximal direction through the connection member 20 (described later), thereby being released from the outer needle hub 3.

The connection member 92 is formed of a resin (resin material). Examples of the material constituting the connection member 92 include various resin materials such as polyolefins such as polyethylene (PE), polypropylene (PP), etc., polycarbonate (PC), polyesters such as polyethylene terephthalate (PET), polybutylene terephthalate (PBT), etc., acrylic resin, ABS resin, AS resin, polyacetal (POM), etc., which may be used either singly or in combination of two or more thereof. With the connection member 92 formed of a resin, the molding or shaping thereof can be carried out easily.

Incidentally, a part of the connection member 92 may be secured to the protector body 91 by such a method as caulking, embedding, fusing, adhesion with an adhesive, etc. Further, the connection member 92 is not limited to the configuration shown in the drawings but may have other shapes or configurations.

In addition, at a distal portion of the protector body 91, the finger holder part (tab) 916 is projectingly formed (is provided). The protector body 91 and the finger holder part 916 are formed integrally with each other. Further, the finger holder part 916 is projecting upward. With the finger holder part 916 pushed in the distal direction by a finger, the outer needle 2 can be moved in the distal direction relative to the inner needle 4.

Incidentally, the protector body 91 and the finger holder part 916 may be formed as separate members and they may be joined to each other. In this case, the material constituting the finger holder part 916 is not particularly limited; for example, materials identical or similar to those mentioned above as materials for the outer needle hub 3 and the inner needle hub 5 can be used. In addition, the finger holder part 916 may be projectingly formed on other part (member), for instance, the outer needle hub 3.

Further, as shown in FIGS. 2, 3 and 5, the indwelling needle assembly 1 has the connection member 20 which has both a function of slip-off preventive means for preventing the protector 9 from slipping off the needle tip 41 of the inner needle 4 when the needle tip 41 is covered by the protector 9 and a function of connection means for connecting the protector 9 with the inner needle hub 5.

The connection member 20 is configured so as to connect the protector body 91 of the protector 9 with the inner needle hub 5. This ensures that when the inner needle hub 5 is moved in the proximal direction, the protector 9 is pulled (moved) in the proximal direction through the connection member 20.

In addition, the connection member 20 is bellows-like in shape and, therefore, can be contracted and expanded. The connection member 20 has such a length that in its maximally expanded state (fully expanded state), the needle tip 41 of the inner needle 4 is located on the proximal side relative to the shutter part 925 of the connection member 92 and the needle tip 41 is stored inside the protector body 91 (is prevented from slipping off the protector body 91).

Thus, the connection member 20 connects the protector body 91 with the inner needle hub 5, and has such a length that in its maximally expanded state the needle tip 41 is contained in the protector body 91. Therefore, the protector 9 is securely prevented from slipping off the needle tip 41 of the inner needle 4, so that the condition where the needle tip 41 is covered by the protector 9 can be maintained assuredly. Accordingly, at the time of discarding the inner needle 4 or the like or in other similar situations, an accident that a worker or the like might stick his or her finger or the like with the needle tip 41 by mistake can be securely prevented from occurring, and high safety is ensured.

Further, the connection member 20 is contracted, or folded, in the assembled state, and is expanded, or spread, in the condition where the inner needle 4 is pulled out of the outer needle 2 and the needle tip 41 is covered by the protector 9.

The connection member 20 as described above is contracted in the assembled state; in the contracted state, the connection member 20 is contained (disposed) in the protector insertion section 51 of the inner needle hub 5 on the proximal side relative to the protector. This ensures that at the time of a puncturing operation, the connection member 20 would not obstruct the operation, so that operability of the indwelling needle assembly 1 is enhanced. Further, there is a merit that a reduction in size of the indwelling needle assembly 1 can be contrived.

In addition, in the contracted state and in the expanded state of the connection member 20, the inner needle 4 is penetrating the connection member 20. This ensures that the inner needle 4 functions as a guide for the connection member 20 at the times of contraction and expansion of the connection member 20. Therefore, for example when the indwelling needle assembly 1 is put into the assembled state (is manufactured), the connection member 20 can be securely prevented from being contracted in an unintended state, specifically, from being contracted without being contained in the inner needle hub 5.

Further, the connection member 20 has a self-restoring property (restoring property) with which it tends to return into its natural state. In the state of being contracted to be shorter than in the natural state, the connection member 20 functions as biasing means for biasing in the expanding direction by its restoring force; on the other hand, in the state of being expanded to be longer than in the natural state, the connection member 20 functions as biasing means for biasing in the contracting direction by its restoring force. Here, the term "natural state" means the state of the connection member 20 in which no external force is exerted on the connection member 20.

Incidentally, the connection member 20 may be configured so as to interconnect the protector 9 and the inner needle 4.

Meanwhile, as shown in FIGS. 2, 3, 5, 7 and 8, the outer needle hub 3 of the indwelling needle assembly 1 is provided with the rib (linear projection) 33 at its proximal portion as a projection engageable with the projecting piece 924 of the connection member 92 of the protector 9.

The rib 33 is formed on the outer peripheral surface of the proximal portion of the outer needle hub 3 along the circumferential direction, and is ring-like in shape in the configuration shown in the figures.

The rib 33 has the sliding surface 331 which is substantially perpendicular to the center axis (axis) O₁ of the inner needle 4 and on which the projecting piece 924 is slidingly moved. When the connection member 92 is changed from the first state shown in FIGS. 3, 4 and 7 to the second state shown in FIGS. 5 and 8, the projecting piece 924 of the connection member 92 is slidingly moved along the sliding surface 331. Specifically, when the inner needle 4 is pulled out of the outer needle 2, the connection member 92 is restored, with its first part 921 released from the outer peripheral surface of the inner needle 4, and, during the restoration of the connection member 92, the projecting piece 924 is slidingly moved along the sliding surface 331 of the rib 33, resulting in the second state.

On the other hand, as mentioned above, both the rib 33 of the outer needle hub 3 (in this embodiment, the whole body of the outer needle hub 3) and a first contact part of the projecting piece 924 of the connection member 92 which makes contact with the sliding surface 331 (in this embodiment, the whole body of the connection member 92) are each formed of a resin (resin material).

This ensures that at the time of pulling the inner needle 4 from the outer needle 2 and releasing the protector 9 from the outer needle hub 3, the projecting piece 924 of the connection member 92 can be prevented from biting into the rib 33, and the projecting piece 924 can be slid (moved) along the sliding surface 331 of the rib 33 smoothly. Consequently, the operation of pulling the inner needle 4 out of the outer needle 2 and releasing the protector 9 from the outer needle hub 3 can be performed smoothly and assuredly.

In addition, as the materials constituting the outer needle hub 3 and the connection member 92, the above-mentioned materials can be used respectively. In a preferred combination of materials, the material constituting the connection member 92 is polyacetal, polycarbonate, polypropylene, polyethylene or the like and the material constituting the outer needle hub 3 is a polyester such as polyethylene terephthalate, polybutylene terephthalate, etc., polypropylene or the like.

Particularly, it is preferable that the resin material constituting the outer needle hub 3 and the resin material constituting the connection member 92 are the same.

Further, it is preferable that b/a is in the range from about 0.3 to 3.0, more preferably about 0.6 to 2.2, where "a" is the Rockwell hardness (according to JIS K 7202) of the rib 33 of the outer needle hub 3, and "b" is the Rockwell hardness of the projecting piece 924 (first contact part) of the connection member 92.

This permits the projecting piece 924 of the connection member 92 to be slidingly moved along the sliding surface 331 of the rib 33 more smoothly.

In addition, the Rockwell hardness of the rib 33 of the outer needle hub 3 is preferably in the range from about 40 to 120, more preferably from about 50 to 110.

Further, the Rockwell hardness of the projecting piece 924 (first contact part) of the connection member 92 is preferably in the range from about 40 to 120, more preferably from about 50 to 110.

In addition, at the time when the inner needle 4 is pulled out of the outer needle 2, the inner needle 4 is slidingly moved on an outer surface of the first part 921 of the connection member 92. As above-mentioned, the inner needle 4 is formed of a metal (metallic material).

However, of the first part 921 of the connection member 92, a second contact part that makes contact with the outer peripheral surface of the inner needle 4 and on which the inner needle 4 is slidingly moved at the time of pulling the inner needle 4 from the outer needle 2 (in this embodiment, the whole part of the connection member 92) is formed of a resin (resin material), as above-mentioned. Therefore, the inner needle 4 can be slidingly moved along the outer surface of the first part 921 smoothly. As a result, the operation of pulling the inner needle 4 out of the outer needle 2 and releasing the protector 9 from the outer needle hub 3 can be carried out smoothly and assuredly.

Incidentally, a lubricant such as a silicone oil, a surfactant, etc. may be given (applied) to either one or both of the sliding surface 331 of the rib 33 of the outer needle hub 3 and the opposed surface 926 (first contact part) of the projecting piece 924 of the connection member 92. This ensures that the frictional resistance (sliding resistance) between the projecting piece 924 and the sliding surface 331 of the rib 33 is reduced, and the projecting piece 924 of the connection member 92 can be slid (moved) along the sliding surface 331 of the rib 33 more smoothly.

In addition, the lubricant is preferably given (applied) also to a part (second contact part) of the outer surface of the first part 921 of the connection member 92 which is brought into contact with the outer peripheral surface of the inner needle 4. This ensures that the frictional resistance between the outer peripheral surface of the inner needle 4 and the outer surface of the first part 921 is reduced, and the inner needle 4 can be slidingly moved along the outer surface of the first part 921 of the connection member 92 more smoothly (the inner needle 4 can be moved relative to the protector 9 more smoothly).

Further, in this embodiment, the projecting piece 924 (first contact part) of the connection member 92 is configured so as to make surface contact with the sliding surface 331 of the rib 33 of the outer needle hub 3. However, it is preferable that the projecting piece 924 (first contact part) is configured so as to make point contact or line contact with the sliding surface 331. This ensures the area (area of contact) of the region of contact between the projecting piece 924 and the sliding surface 331 of the rib 33 is made small, and the frictional resistance between the projecting piece 924 and the sliding surface 331 of the rib 33 is reduced. Accordingly, the projecting piece 924 can be slidingly moved along the sliding surface 331 of the rib 33 more smoothly. These configuration examples will be described below.

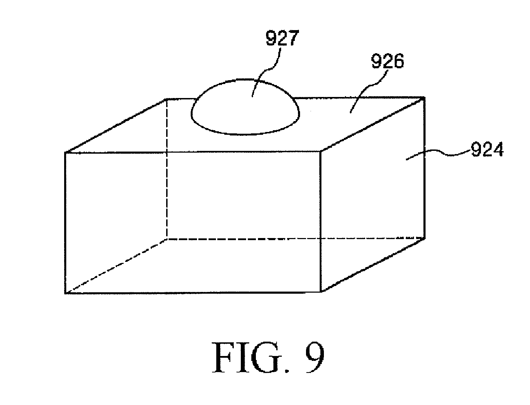

In a configuration example shown in FIG. 9, on the opposed surface 926 of the projecting piece 924 of the connection member 92, a rounded projection 927 is formed as the first contact part. This ensures that the projecting piece 924 makes point contact with the sliding surface 331 of the rib 33.

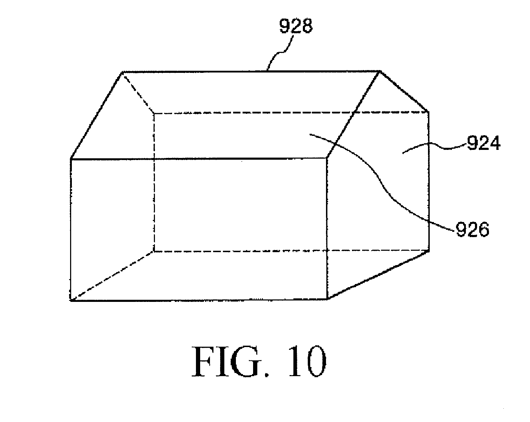

In a configuration example shown in FIG. 10, at the opposed surface 926 of the projecting piece 924 of the connection member 92, a rectilinear angular part 928 is formed as the first contact part. This ensures that the projecting piece 924 makes line contact with the sliding surface 331 of the rib 33. Incidentally, the projecting piece 924 is moved in the direction of the straight line of the angular part 928, by the restoration of the connection member 92.

Further, in this embodiment, the sliding surface 331 of the rib 33 is substantially perpendicular to the center axis O₁ of the inner needle 4, but it is preferable that the sliding surface 331 of the rib 33 is tilted relative to the center axis O₁ of the inner needle 4. This ensures that at the time of pulling the inner needle 4 out of the outer needle 2 and releasing the protector 9 from the outer needle hub 3, the resistance (resistance to release) can be reduced. As a result, the operation of pulling the inner needle 4 out of the outer needle 2 and releasing the protector 9 from the outer needle hub 3 can be carried out more smoothly and assuredly. In addition, since the sliding surface 331 is tilted, the area (area of contact) of the region of contact between the projecting piece 924 and the sliding surface 331 of the rib 33 is small, so that the frictional resistance between the projecting piece 924 and the sliding surface 331 of the rib 33 is small. Consequently, the projecting piece 924 can be slidingly moved along the sliding surface 331 of the rib 33 more smoothly.

One example of the method of using the indwelling needle assembly 1 (in the case of puncturing a blood vessel) (operation) will be described in detail below.
[1] The indwelling needle assembly 1 is put into the assembled state (see FIGS. 1, 3, 4 and 7), and a connector attached to an end portion of an infusion line is preliminarily connected to the connector 72 so that an infusion liquid from the infusion line can be supplied.
   Incidentally, in this case, a predetermined part on the tube 7 or the infusion line is pinched, for example, by a clamp (an example of flow path opening/closing means) so as to close the lumen of the tube 7 or the infusion line.
[2] Next, the closure of the tube 7 or the infusion line with the clamp or the like is released, and the infusion liquid from the infusion line is introduced through the tube 7 into the outer needle hub 3.
   The infusion liquid introduced into the outer needle hub 3 fills up the branch flow path 32 and the flow path 31 on the distal side relative to the seal member, and is introduced into the lumen 21 of the outer needle 2, whereby the lumen 21 of the outer needle 2 is primed with the infusion liquid. In this instance, part of the infusion liquid flows out via the tip opening 22 of the outer needle 2.
[3] After the priming is completed in this manner, the tube 7 or the infusion line is again closed with a clamp or the like, then the inner needle hub 5 is grasped, and the outer needle 2 and the inner needle 4 integrated to each other are caused to puncture a blood vessel (a vein or an artery) of a patient.
   When the blood vessel is captured by the outer needle 2, the internal pressure in the blood vessel (blood pressure) causes blood to flow back in the proximal direction through the inner needle 4 and then through the lumen 21 of the outer needle 2, so that this blood flow can be confirmed by at least one part of the outer needle 2, the outer needle hub 3, the inner needle hub 5 and the tube 7 that has inside visibility.
   After the confirmation of the blood flow, the outer needle 2 is advanced by a very short distance in the distal direction along the inner needle 4, using the inner needle 4 as a guide.
   In addition, at the time of puncturing the blood vessel in this manner, the lumen 21 of the outer needle 2 has already been primed with the infusion liquid, so that erroneous penetration of a bubble or bubbles into the blood vessel is securely prevented, and safety is extremely high.
[4] When the blood vessel has been captured by the outer needle 2 (when the outer needle 2 has been moved to a target position), the outer needle 2 or the outer needle hub 3 is fixed by one hand, and the inner needle hub 5 is gripped by the other hand and pulled in the proximal direction. By this, operations (motions) ranging from a motion of pulling the inner needle 4 out of the outer needle 2 to the release of the protector 9 from the outer needle hub 3 are carried out sequentially and continuously. Specifically, first, the inner needle 4 is moved in the proximal direction and pulled out of the outer needle 2.
[5] When the inner needle 4 has been moved further in the proximal direction and the needle tip 41 has passed through the slit, the seal member having the self-closing property closes the slit by its own elastic force. This ensures that leakage of liquid through the slit is prevented from occurring, and asepsis of the inside of the outer needle hub 3 and the infusion line is secured.
[6] When the inner needle 4 is moved further in the proximal direction and the needle tip 41 reaches the proximal side of the first part 921 of the connection member 92, the connection member 92 is opened (restored) by its own elastic force (restoring force), and is moved by being pulled also in the proximal direction through the connection member 20, resulting in the second state, as shown in FIGS. 5 and 8. After the connection member 92 is thus put into the second state, even if the needle tip 41 of the inner needle 4 tends to move so as to return in the distal direction again, the needle tip 41 abuts on the shutter part 925 of the connection member 92 and, therefore, cannot return.
[7] The inner needle hub 5 is moved further in the proximal direction, whereby the protector 9 is moved by being pulled in the proximal direction through the connection member 20, and is separated (released) from the outer needle hub 3.
   The connection member 20 has such a length that, in its maximally expanded state, the needle tip 41 is contained in the protector body 91. Therefore, the protector 9 can be securely prevented from slipping off the needle tip 41. Accordingly, the condition where the needle tip 41 is covered with the protector 9 can be maintained assuredly.
   After the inner needle 4 is pulled out of the outer needle 2 in this manner, the inner needle 4 and the inner needle hub 5 are useless, and, therefore, they are put to a discarding procedure.
   Of the inner needle 4, the needle tip 41 is covered by the protector 9. Particularly, the needle tip 41 is prevented from moving toward the distal side beyond the shutter part 925 of the connection member 92 to protrude from the distal end of the protector 9. Therefore, an accident that a person in charge of the discarding treatment or the like might pierce his or her finger or the like with the needle tip 41 by mistake is prevented from occurring.
[8] Subsequently, the wings 12a and 12b are fixed to a skin with a pressure sensitive adhesive tape or the like, the closure of the tube 7 or the infusion line with the clamp or the like is released, and supply of the infusion liquid is started.
   The infusion liquid supplied from the infusion line is injected into the patient's blood vessel via the respective lumens or inner cavities of the connector 72, the tube 7, the outer needle hub 3 and the outer needle 2.
   As has been described above, according to this indwelling needle assembly 1, at the time of pulling the inner needle 4 out of the outer needle 2 and releasing the protector 9 from the outer needle hub 3, the projecting piece 924 of the connection member 92 can be prevented from biting into the rib 33, and the projecting piece 924 can be slidingly moved along the sliding surface 331 of the rib 33 smoothly. Therefore, the operation of pulling the inner needle 4 out of the outer needle 2 and releasing the protector 9 from the outer needle hub 3 can be carried out smoothly and assuredly.
   Incidentally, in the present invention, it suffices that, of the connection member 92, only the first contact part as the part for making contact with the sliding surface 331 of the rib 33 of the outer needle hub 3 is formed of a resin. Thus, the other parts than the first contact part, of the connection member 92, may be formed of other material than the resin material.
   In addition, in the present invention, it is sufficient if, of the outer needle hub 3, only the rib (projection) 33 is formed of a resin. Thus, other parts than the rib 33 of the outer needle hub 3, may be formed of other material than the resin.

### <Second Embodiment>

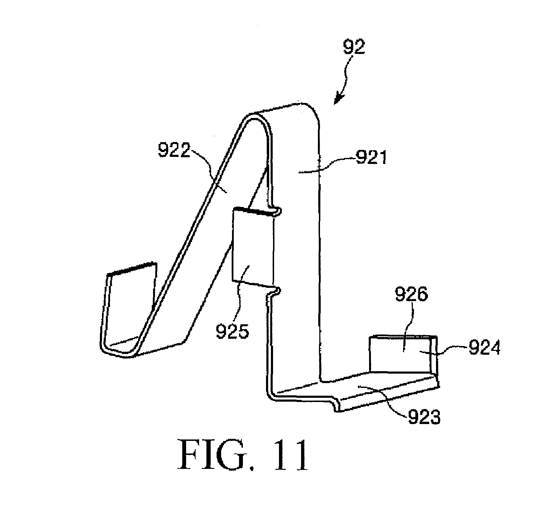

FIG. 11 is a perspective view showing a connection member of a protector in a second embodiment of the indwelling needle assembly according to the present invention.

The second embodiment will be described below, referring mainly to differences from the above-described first embodiment, and descriptions of the same items as above will be omitted.

As shown in FIG. 11, in the indwelling needle assembly 1 according to the second embodiment, of a connection member 92, only a projecting piece (projecting part) 924 is formed of a resin, and the other parts are formed of other material than the resin.

The connection member 92 can be produced, for example, such that other part (structure) than the projecting piece 924 is formed by bending an elastic (elastically deformable) belt-like plate member, and the projecting piece 924 is secured (joined) to the structure by adhesion, fusing or the like.

In addition, examples of the material constituting the other parts than the projecting piece 924 of the connection member 92 include various metallic materials such as, for example, stainless steel, aluminum or aluminum alloys, titanium or titanium alloys, copper or copper alloys, etc.

According to this indwelling needle assembly 1, effects equivalent to those of the indwelling needle assembly 1 in the first embodiment described above can be obtained.

Incidentally, the second embodiment can be applied also to a fourth embodiment which will be described later.

### <Third Embodiment>

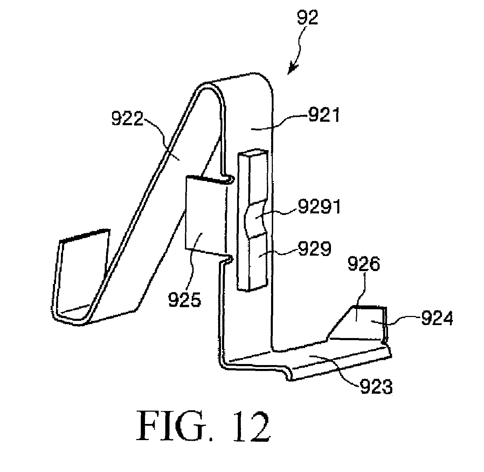

FIG. 12 is a perspective view showing a connection member of a protector in a third embodiment of the indwelling needle assembly according to the present invention.

The third embodiment will be described below, referring mainly to differences from the above-described first embodiment, and descriptions of the same items as above will be omitted.

As shown in FIG. 12, in the indwelling needle assembly 1 according to the third embodiment, of a connection member 92, only a second contact part 929, which makes contact with an outer peripheral surface of an inner needle 4 and on which the inner needle 4 is slidingly moved at the time of pulling the inner needle 4 out of the outer needle 2, is formed of a resin, and the other parts are formed of other material than the resin.

The connection member 92 can be produced, for example, such that other part (structure) than the second contact part 929 is formed by bending an elastic (elastically deformable) belt-like plate member, and the second contact part 929 is secured (joined) to the structure by adhesion, fusing or the like.

Preferably, the second contact part 929 is secured to an outer surface (a surface on the outside) of a first part 921 constituting one portion of V-shaped part of the connection member 92, whereas the second contact part 929 is formed with a groove (recess) 9291 for guiding the inner needle 4.

In addition, examples of the material constituting the other parts than the second contact part 929 of the connection member 92 include various metallic materials such as, for example, stainless steel, aluminum or aluminum alloys, titanium or titanium alloys, copper or copper alloys, etc.

Further, in this embodiment, further, it is preferable that the projecting piece (projecting part) 924 is also formed of a resin.

According to this indwelling needle assembly 1, effects equivalent to those of the indwelling needle assembly 1 in the first embodiment described above can be obtained.

Incidentally, the third embodiment can be applied also to the fourth embodiment which will be described later.

### <Fourth Embodiment>

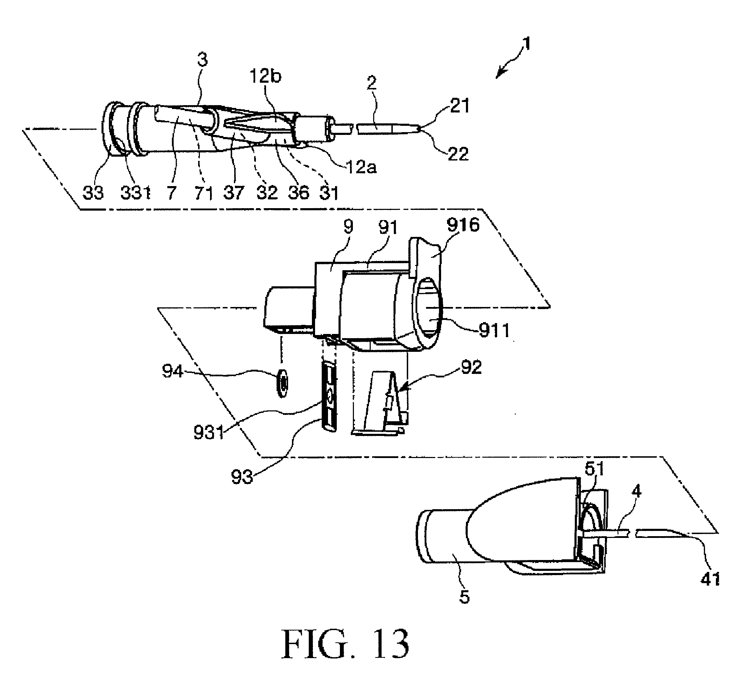

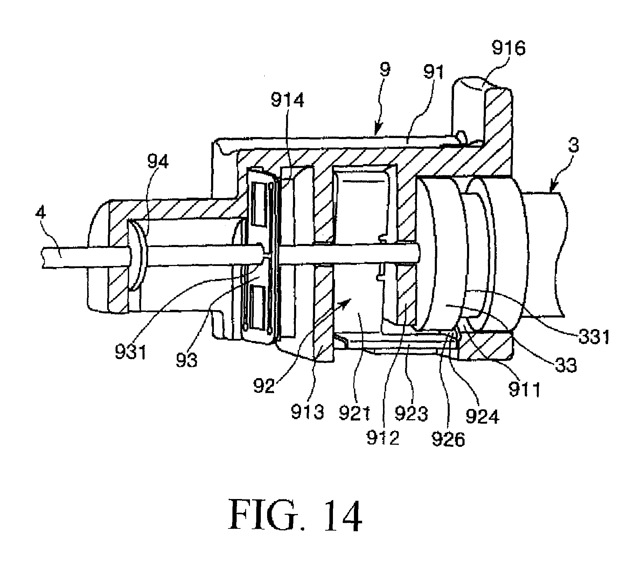

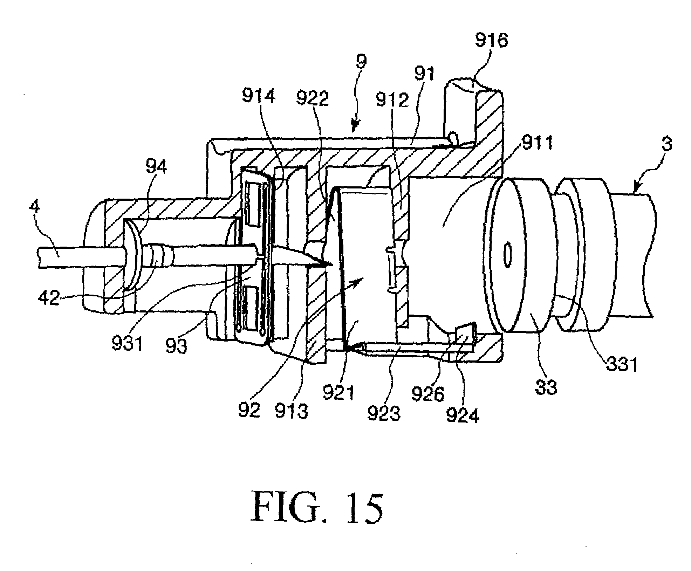

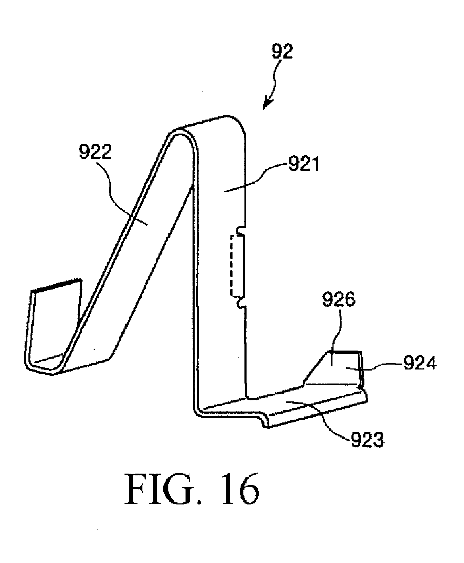

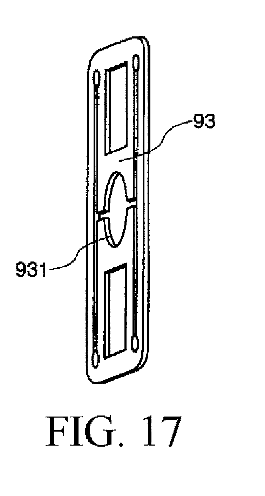

FIG. 13 is an exploded perspective view showing a fourth embodiment of the indwelling needle assembly according to the present invention; FIGS. 14 and 15 are each sectional views showing an outer needle hub, an inner needle, a protector, etc. of the indwelling needle assembly shown in FIG. 13; FIG. 16 is a perspective view showing a connection member of the protector in the indwelling needle assembly shown in FIG. 13; and FIG. 17 is a perspective view showing a shutter member of the protector in the indwelling needle assembly shown in FIG. 13.

Incidentally, in the following description, the left side in FIGS. 13, 14 and 15 will be referred to as a "proximal" side and the right side as a "distal" side.

The fourth embodiment will be described below, referring mainly to differences from the above-described first embodiment, and descriptions of the same items as above will be omitted.

As shown in FIGS. 13 to 17, in the indwelling needle assembly 1 according to the fourth embodiment, a protector 9 has a protector body 91, and a connection member 92 and a shutter member 93 and a ring 94 which are provided inside the protector body 91. In addition, an inner needle 4 is provided with a large diameter part (enlarged diameter part) 42 on the distal side thereof. The large diameter part 42 is large in outside diameter (enlarged in outside diameter). Further, the connection member 92 is not provided with any shutter part 925, and a connection member 20 is omitted.

Specifically, as shown in FIGS. 14 and 15, a wall section 914 is formed on the proximal side of a wall section 913 of the protector body 91, and the shutter member 93 is provided on the proximal side of the wall section 914.

As shown in FIG. 17, the shutter member 93 is composed of an elastic plate member, and is provided in its central portion with a hole 931 into which the inner needle 4 is inserted in the assembled state. In addition, the shutter member 93 is formed with a plurality of cutouts, which permits the shutter member 93 to be deformed easily.

The diameter of the hole 931 when the shutter member 93 is in a natural state is set to be smaller than the diameter of the large diameter part 42 of the inner needle 4 and be larger than the diameter of the other parts of the inner needle 4. This ensures that when the shutter member 93 is in the natural state, the large diameter part 42 cannot pass through the hole 931.

In addition, the shutter member 93 is configured such that when the inner needle 4 is moved in the proximal direction and the large diameter part 42 of the inner needle 4 is about to pass through the hole 931, starting from the assembled state in which the large diameter part 42 of the inner needle 4 is located on the distal side of the shutter member 93, the shutter member 93 is deformed so that the diameter of the hole 931 becomes greater than the diameter of the large diameter part 42. This permits the large diameter part 42 to pass through the hole 931.

On the other hand, when the inner needle 4 is moved in the distal direction and the large diameter part 42 of the inner needle 4 is about to pass through the hole 931, starting from the condition in which the large diameter part 42 is located on the proximal side of the shutter member 93, the shutter member 93 is inhibited from deformation (is maintained in the natural state) by the wall section 914. This prevents the large diameter part 42 from passing through the hole 931.

Consequently, the needle tip 41 is inhibited from protruding from the distal end of the protector 9.

Further, the ring 94 is disposed on the proximal side of the protector body 91 (on the proximal side relative to the shutter member 93). The diameter of the hole in the ring 94 is set to be smaller than the diameter of the large diameter part 42 of the inner needle 4 and be greater than the diameter of the other parts of the inner needle 4. This prevents the large diameter part 42 to pass through the hole of the ring 94. As a result, the protector 9 is securely prevented from slipping off the needle tip 41 of the inner needle 4, and the protector 9 and the inner needle 4 are connected with each other.

Thus, the ring 94 has both a function of slip-off preventive means for preventing the protector 9 from slipping off the needle tip 41 of the inner needle 4 when the needle tip 41 is covered by the protector 9 and a function of connection means for connecting the protector 9 and the inner needle 4 to each other.

According to the present indwelling needle assembly 1, effects equivalent to those of the indwelling needle assembly 1 in the first embodiment described above can be obtained.

While the indwelling needle assembly according to the present invention has been described above based on the embodiments shown in the drawings, the invention is not limited to the embodiments, and the parts constituting the indwelling needle assembly can be replaced by parts of arbitrary configurations which can exhibit functions equivalent to the original. Further, arbitrary components or structures may be added.

In addition, the present invention may be embodied by a combination of arbitrary two or more configurations (features) of the above-described embodiments.

Further, the indwelling needle assembly according to the present invention is not limited to those for used in the state of being inserted in a blood vessel; for example, the indwelling needle assembly of the invention is also applicable to those for use in the state of being inserted in an abdominal cavity, a thoracic cavity, a lymph vessel, a vertebral canal or the like.

In addition, in the present invention, the shape of the slit in the seal member is not limited to the shape of a straight line segment; for example, the shapes of a cross, capital Y, capital T, capital H, etc. may also be adopted.

Further, in the present invention, the protector is not limited to those of the configurations shown in the drawings, and may be any one that can be detachably connected to the outer needle hub. Particularly, protectors of various configurations can be used insofar as they cover at least the needle tip of the inner needle when the inner needle has been pulled out of the outer needle.

### INDUSTRIAL APPLICABILITY

The indwelling needle assembly according to the present invention includes: an inner needle having a sharp needle tip at a distal end; an inner needle hub secured to a proximal portion of the inner needle; a hollow outer needle into which the inner needle is inserted; an outer needle hub which is secured to a proximal portion of the outer needle and which has a resin projection at a proximal portion thereof; and a protector which has an elastic connection member with a projecting part engageable with the projection, and which is detachably connected to the outer needle hub by engagement of the projecting part with the projection; wherein the projection of the outer needle hub includes a sliding surface on which the projecting part is slidingly moved; the connection member takes a first state in which the projecting part is engaged with the projection and a second state in which the projecting part is disengaged from the projection, and, when the connection member is changed from the first state to the second state, the projecting part is slidingly moved along the sliding surface of the projection; and a first contact part of the projecting part which is brought into contact with the sliding surface is formed of a resin. Therefore, the protector can be released from the outer needle hub smoothly. Accordingly, the indwelling needle assembly according to the present invention has industrial applicability.

## Claims

1. An indwelling needle assembly comprising:
an inner needle having a sharp needle tip at a distal end;
an inner needle hub secured to a proximal portion of the inner needle;
a hollow outer needle into which the inner needle is inserted;
an outer needle hub which is secured to a proximal portion of the outer needle and which has a resin projection at a proximal portion thereof; and
a protector which has an elastic connection member with a projecting part engageable with the projection, and which is detachably connected to the outer needle hub by engagement of the projecting part with the projection, wherein
the projection of the outer needle hub includes a sliding surface on which the projecting part is slidingly moved,
the connection member takes a first state in which the projecting part is engaged with the projection and a second state in which the projecting part is disengaged from the projection, and, when the connection member is changed from the first state to the second state, the projecting part is slidingly moved along the sliding surface of the projection, and
a first contact part of the projecting part which is brought into contact with the sliding surface is formed of a resin.

2. The indwelling needle assembly according to claim 1, wherein the first contact part is brought into point contact or line contact with the sliding surface.

3. The indwelling needle assembly according to claim 1, wherein b/a is in a range from 0.3 to 3, where "a" is the Rockwell hardness of the projection and "b" is the Rockwell hardness of the first contact part.

4. The indwelling needle assembly according to claim 1, wherein the connection member is held in the first state in a state of being elastically deformed by making contact with an outer peripheral surface of the inner needle, the connection member is restored by parting from the outer peripheral surface of the inner needle when the inner needle is pulled out of the outer needle, and, when the connection member is restored, the projecting part is slidingly moved along the sliding surface of the projection, resulting in the second state.

5. The indwelling needle assembly according to claim 1, wherein
the connection member has a second contact part which makes contact with the inner needle and on which the inner needle is slidingly moved when the inner needle is pulled out of the outer needle; and
the second contact part is formed of a resin.

6. The indwelling needle assembly according to claim 5, wherein the connection member has a shape obtained by bending a belt-like member, and the second contact part is secured to one outer surface of the connection member.

7. The indwelling needle assembly according to claim 1, wherein the whole connection member is formed of a resin.

8. The indwelling needle assembly according to claim 1, wherein
a direction in which the projecting part is moved by restoration of the connection member is a direction substantially perpendicular to an axis of the inner needle; and
the projection is formed at an outer peripheral surface of the outer needle hub along a circumferential direction of the outer needle hub.

9. The indwelling needle assembly according to claim 1, further comprising connection means for connecting the inner needle or the inner needle hub with the protector, wherein
when the inner needle is pulled out of the outer needle, the inner needle hub is moved in a proximal direction, whereby the protector is moved in the proximal direction so as to be released from the outer needle hub.

10. The indwelling needle assembly according to claim 1, wherein the protector covers at least the needle tip of the inner needle when the inner needle has been pulled out of the outer needle.
